(19) **Europäisches Patentamt / European Patent Office / Office européen des brevets**

(11) **EP 2 680 826 B1**

(12) **EUROPEAN PATENT SPECIFICATION**

(45) Date of publication and mention
of the grant of the patent:
**23.08.2017 Bulletin 2017/34**

(21) Application number: **12712144.0**

(22) Date of filing: **27.02.2012**

(51) Int Cl.:
*A61K 9/48* (2006.01)     *A61K 31/593* (2006.01)
*A61P 3/02* (2006.01)

(86) International application number:
**PCT/GB2012/050434**

(87) International publication number:
**WO 2012/117236 (07.09.2012 Gazette 2012/36)**

(54) **VITAMIN D COMPOSITION**

VITAMIN D ZUSAMMENSETZUNG

COMPOSITION DE VITAMINE D

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **02.03.2011 GB 201103519
19.05.2011 GB 201108393**

(43) Date of publication of application:
**08.01.2014 Bulletin 2014/02**

(73) Proprietor: **D3 Pharma Limited
Kent DA14 6NE (GB)**

(72) Inventor: **HUATAN, Hiep
Sidcup
Kent DA14 6NE (GB)**

(74) Representative: **Docherty, Robert Charles et al
Symbiosis IP Limited
NAFIC
Office 14FA05
Sand Hutton
York, North Yorkshire YO41 1LZ (GB)**

(56) References cited:
WO-A1-03/059358     WO-A1-2006/116204
WO-A1-2008/134512     WO-A1-2010/111397

• X. Groshens ET AL: "Hypromellose Hard
Capsules - Reproducibility of the In Vitro
Dissolution Performance in USP SGF Media",
Capsugel, 1 July 2010 (2010-07-01), pages 1-4,
XP55034505, Retrieved from the Internet:
URL:http://capsugel.com/media/library/hypr
omellose-hard-capsules-reproducibility-of-
the-in-vitro-dissolution-performance-in-us
p-sgf-media.pdf [retrieved on 2012-08-02]
• I. CHIWELE ET AL: "The Shell Dissolution of
Various Empty Hard Capsules", CHEM. PHARM.
BULL., vol. 48, no. 7, 1 July 2000 (2000-07-01),
pages 951-956, XP55034504,

EP 2 680 826 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

The present disclosure relates to high dosage vitamin $D_3$ formulations that can be used for the treatment of vitamin D deficiency and insufficiency, and for the prevention or treatment of various medical conditions resulting from a deficient or insufficient vitamin D state.

It has long been known that vitamin D is a key steroid hormone that regulates calcium homeostasis. Vitamin D is a fat soluble vitamin that is found in food and is also made in the body after exposure to ultraviolet (UV) rays from the sun. Sunshine is a significant source of vitamin D because UV rays from sunlight trigger vitamin D synthesis in the skin. Vitamin D exists in several forms, each with a different level of activity. The two most active forms of vitamin D are Ergocalciferol ($D_2$) and Cholecaliferol ($D_3$). Vitamin $D_3$ is more active than vitamin $D_2$ and in addition, it has a longer biological half-life which makes it the preferred form for clinical and therapeutic applications (Armas et al., JCEM, 98(11), 2004). The collective term for these vitamin D forms is calciferol. Other forms are relative inactive in the body. Once vitamin D is produced in the skin or consumed in food, it is converted in the liver to a pre-hormone (25-hydroxyvitamin D [25(OHD)]) which is also referred to calcifediol. 25(OHD) is then converted in the kidneys to form 1,25 dihydroxyvitamin D, the physiologically active form of vitamin D. Active vitamin D functions as a hormone because it sends a message to the intestines to increase the absorption of calcium and phosphorus. It is accepted internationally that the serum level of 25(OHD), the pre-cursor of the active form, 1,25 dihydroxyvitamin D, is the most accurate indicator for vitamin D status in humans.

It is well established that the major function of vitamin D is to maintain normal blood levels of calcium and phosphorus. By promoting calcium absorption, vitamin D helps to form and maintain strong bones. Vitamin D also works in concert with a number of other vitamins, minerals and hormones to promote bone mineralization. Without vitamin D, bones can become thin, brittle, or misshapen. In adults, a lack of vitamin D results in the development of osteoporosis, osteomalacia and increased risk of hip (and general bone) fracture. In children, a lack of vitamin D leads to the development of rickets, a softening of bones that can rapidly progress to skeletal fractures and deformity. There is an association between low levels of vitamin D with a wide spectrum of serious diseases, including cancer, multiple sclerosis, type 1 diabetes, heart disease, high blood pressure and schizophrenia.

The dose for vitamin D is commonly expressed by weight (typically in micrograms) or by international unit (IU), which is a measure of biological activity. One IU is equivalent to 0.025 microgram of vitamin D ($D_2$ or $D_3$).

For people who are already deficient or insufficient in vitamin D i.e., serum levels less than 50nmoles/L, the dose of vitamin D required to correct this deficiency/insufficiency status is much higher, typically in the range of 100,000 IU (per month) or up to 300,000 IU every three months. Despite the well established practice for high dosage vitamin D deficiency treatment, there are limited means for enabling the treatment regime in a convenient and compliant manner. This practice commonly leads to poor compliance as it is not ideal to dose (often elderly or paediatric patients) with such a large number of unit doses.

A further challenge which exists is that vitamin D is very light sensitive and is subject to oxidative degradation. The oxidation kinetics i.e., the rate of reaction increases with concentration, hence high dosage (more concentrated) vitamin D products have a greater propensity for oxidation compared with lower dosage (less concentrated) formulations. Moreover, high dosage vitamin D products require stabilisation by means of additives and/or oxygen protective barrier. Typically, soft or hard gelatinised capsules are used as they offer high oxygen barrier properties for vitamin D and oil carrier therein. The key issue with gelatin-based material is that it can only be sourced from animal or fish, which renders it unsuitable for many people, especially vegetarians and/or those who do not consume meat/fish through religious or cultural believes.

Vitamin D is a hydrophobic molecule and therefore generally has poor water solubility. This is also the case with respect to vitamins A, E, K, B8, B9, B12, and B2 which collectively are referred to as fat soluble vitamins. There have been attempts to improve the solubility of fat soluble vitamins. These approaches include the synthesis of vitamin analogues with improved solubility and formulations that increase the solubility of selected vitamins. For example, WO2009/098295 discloses a pulverulent [powdered] composition which has improved aqueous solubility for selected vitamins such as vitamin $D_2$ and $D_3$. WO2009/009134 discloses a composition for solubilising vitamins comprising a silicone and a solvent and vitamins such as retinoids, vitamin A esters and vitamin D and analogues of vitamin D. Nottoli and Davis (US 2009/0196862A1) discloses preparation containing vitamin $D_3$ (cholecalciferol) to provide general health benefits and with potential for use in the treatment of a number of medical conditions. Chen et al. (US 2003/0191093A1) discloses pharmaceutical compositions comprising an active vitamin D compound in emulsion pre-concentrate formulations, as well as emulsions and submicron droplet emulsions produced therefrom. WO 2010/111397 discloses hard capsules filled with vitamin D. There thus remains a long felt need for high dosage vitamin D preparations that can deliver therapeutic doses of vitamin D in a pharmaceutically acceptable form for the rapid repletion and maintenance of vitamin D levels in people with vitamin D deficiency or insufficiency. Such preparations can further be used to treat or prevent of a variety of health conditions that are affected by vitamin D, directly or indirectly.

According to an aspect of the invention there is provided a pharmaceutical composition adapted for oral delivery comprising: at least 0.5mg [20,000 IU] of vitamin $D_3$, a mixture of caprylic/capric triglyceride excipient that stabilizes vitamin $D_3$, butylated hydroxyanisole, colloidal silicon dioxide and an outer capsule consisting of cellulosic polymer derivatives wherein vitamin $D_3$ is 0.25-1.0% w/w of the composition.

We disclose a pharmaceutical composition adapted for oral delivery comprising: at least 20,000 IU of vitamin D, a lipid based carrier excipient that stabilizes vitamin D and an outer capsule or layer consisting essentially of a non-animal derived polymer or polymers.

We disclose that vitamin D is vitamin $D_3$.

In a preferred embodiment of the invention vitamin $D_3$ is provided at 0.5mg [20,000 IU], 1.0mg [40,000IU], 1.25mg [50,000 IU], 1.5mg [60,000IU], 1.75mg [70,000 IU], 2mg [80,000IU], 2.25mg [90,000 IU] or 2.5mg [100,000IU] vitamin $D_3$

In a preferred embodiment of the invention vitamin $D_3$ is provided at 20,000-100,000 IU; preferably at least 40,000 IU vitamin D or 50,000, 60,000, 70,000, 80,000 or 90,000 IU. Preferably vitamin $D_3$ is provided at around 100,000 IU.

We disclose a lipid based carrier excipient comprising an oil or a mixture of oils.

We disclose that said oil is plant derived oil.

We disclose plant derived oil selected from the group consisting of: almond oil, arachis oil, canola oil, cod liver oil, corn oil, cotton seed oil, flaxseed oil, grape seed oil, peanut oil, safflower oil, sesame oil, soybean oil, sunflower oil, walnut oil, coconut oil or palm kernel oil.

We disclose plant oil comprising coconut oil and palm kernel oil.

We disclose that oil includes at least one or a combination of fatty acids selected from the group consisting of: caprylic/capric triglycerides (for example Miglyol® 810, 812 and 812N), caprylic/capric/linoleic triglycerides (for example Miglyol® 818), caprylic/capric/myristic/stearic fatty acid triglycerides (for example Softisan® 378), capylic/capric/succinic fatty acid triglycerides (for example Miglyol® 829), caprylic/capric triglycerides with stearalkonium benonite and propylene carbonate (for example Migyol® Gel T), caprylic/capric triglycerides with stearalkonium hectorite and propylene carbonate (for example Migyol® Gel B).

Preferably said oil according to the invention is conveniently provided as Miglyol® 812N.

Miglyol® 812N is a mixture of caprylic/capric fatty acids and in addition to solubilising vitamin D it has anti-oxidant properties conferring oxidative protection.

We disclose that the composition includes an anti-oxidant.

Preferably said anti-oxidant is butylated hydroxyanisole.

We disclose a composition consisting of essentially of: 0.25-1.0% w/w vitamin $D_3$, 98.95-99.7% w/w Miglyol 812N and 0.02-0.08% w/w, butylated hydroxyanisole or butylated hydroxytoluene wherein said composition is contained in a HPMC capsule.

We disclose a composition consisting of 0.25% w/w vitamin $D_3$, 99.7% w/w Miglyol 812N and 0.05% w/w, butylated hydroxyanisole wherein said composition is contained in a HPMC capsule.

We disclose a composition consisting of: 1.0% w/w vitamin $D_3$, 98.5% w/w Miglyol 812N and 0.05% w/w, butylated hydroxyanisole wherein said composition is contained in a HPMC capsule.

We disclose the composition contained in a HPMC capsule wherein the capsule consists essentially of: 0.5mg vitamin $D_3$, 199.4mg Miglyol 812N and 0. 1mg butylated hydroxyanisole.

We disclose the composition is contained in a HPMC capsule wherein the capsule consists essentially of: 1.25mg vitamin $D_3$, 498.5mg Miglyol 812N and 0.25mg butylated hydroxyanisole.

We disclose the composition is contained in a HPMC capsule wherein the capsule consists essentially of: 2.5mg vitamin $D_3$, 997mg Miglyol 812N and 0.5mg butylated hydroxyanisole.

We disclose the composition is contained in a HPMC capsule wherein the capsule consists essentially of: 1.25mg vitamin $D_3$, 248.62mg Miglyol 812N and 0.13mg butylated hydroxyanisole.

We disclose the composition is contained in an HPMC capsule wherein the capsule consists essentially of: 2.5mg vitamin $D_3$, 247.37mg Miglyol 812N and 0.13mg butylated hydroxyanisole.

We disclose that the lipid based carrier excipient is a wax.

We disclose the lipid based carrier excipient is a Poloxamer; preferably Poloxamer 188.

We disclose the composition comprises an outer capsule shell or layer that comprises a plant derived polymer or polymer mix.

Preferably said plant derived polymer is cellulose based; for example hydroxypropylmethyl cellulose (HMPC) or other cellulosic polymer derivatives.

We disclose the composition consists essentially of 0.25-1.00% w/w vitamin $D_3$ and 99-99.8% w/w Poloxamer 188 wherein said composition is contained in a HPMC capsule.

We disclose the composition consists of 0.26% w/w vitamin $D_3$ and 99.74% w/w Poloxamer 188 wherein said composition is contained in a HPMC capsule.

We disclose the composition consists of 1.00% w/w vitamin $D_3$ and 99% w/w Poloxamer 188 wherein said composition is contained in a HPMC capsule.

We disclose that the composition is contained in a HPMC capsule wherein the capsule consists essentially of 0.5mg vitamin $D_3$ and 189.5mg Poloxamer 188.

We disclose that the composition is contained in a HPMC capsule wherein the capsule consists essentially of 1.25mg vitamin $D_3$ and 473.75mg Poloxamer 188.

We disclose that the composition is contained in a HPMC capsule wherein the capsule consists essentially of 2.5mg vitamin $D_3$ and 947.5mg Poloxamer 188.

We disclose that the composition is contained in a HPMC capsule wherein the capsule consists essentially of 1.25mg vitamin $D_3$ and 248.75mg Poloxamer 188.

We disclose that the composition is contained in a HPMC capsule wherein the capsule consists essentially of 2.5mg vitamin $D_3$ and 247.5mg Poloxamer 188.

We disclose that the composition further includes at least one viscosity modifying agent.

A viscosity modifying agent is a pharmaceutically acceptable additive alters the viscosity of the formulation vehicle to aid processing and capsule filling.

Preferably said viscosity modifying agent is colloidal silicon dioxide, for example Aerosil® 200.

In a preferred embodiment of the invention said composition comprises 0.5-5.0% w/w colloidal silicon dioxide e.g. Aerosil® 200.

Preferably said composition comprises 2.0-4.0%w/w colloidal silicon dioxide e.g. Aerosil® 200.

In an preferred embodiment of the invention said composition is contained in an HPMC capsule wherein said capsule consists essentially of 0.5 mg vitamin $D_3$, 199.40mg Miglyol 812N, 0.10mg butylated hydroxyanisole and 5.0mg Aerosil® 200.

In an alternative preferred embodiment of the invention said composition consists of 0.24% w/w vitamin $D_3$, 97.27% w/w Miglyol 812N, 0.05% w/w butylated hydroxyanisole, and 2.44% w/w Aerosil® 200 wherein said composition is contained in an HPMC capsule.

In a preferred embodiment of the invention said composition is contained in an HPMC capsule wherein said capsule consists essentially of 2.5mg vitamin $D_3$, 277.36mg Miglyol 812N, 0.14mg butylated hydroxyanisole and 7mg Aerosil® 200.

In an alternative preferred embodiment of the invention said composition is consists of 0.87%w/w vitamin $D_3$, 96.64%w/w Miglyol 812N, 0.05% w/w butylated hydroxyanisole and 2.44% w/w Aerosil® 200 contained in an HPMC capsule.

The compositions of the invention are administered in effective amounts. An "effective amount" is that amount of a composition that alone, or together with further doses, produces the desired response. Such amounts will depend, of course, on the particular condition being treated, the severity of the condition, the individual patient parameters including age, physical condition, size and weight, the duration of the treatment, the nature of concurrent therapy (if any), and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

In the case of treating vitamin $D_3$ deficiency the desired response is inhibiting the progression of disease and returning the subject to a physiological normal state. This may involve only slowing the progression of the disease temporarily, although more preferably, it involves halting the progression of the disease permanently.

The pharmaceutical compositions used in the foregoing methods preferably are non-sterile and contain an effective amount of vitamin $D_3$ for producing the desired response in a unit of weight or volume suitable for oral administration to a patient. The response can, for example, be measured by determining decrease of disease symptoms.

The doses of vitamin $D_3$ administered to a subject can be chosen in accordance with different parameters. Factors include the desired period of treatment. In the event that a response in a subject is insufficient at the initial doses applied, higher doses (or effectively higher doses by a different, more localized delivery route) may be employed to the extent that patient tolerance permits.

When administered, the vitamin $D_3$ compositions are applied in pharmaceutically-acceptable amounts and in pharmaceutically-acceptable compositions. The term "pharmaceutically acceptable" means physiologically or toxicologically tolerable. Such preparations may routinely contain salts, buffering agents, preservatives, compatible carriers, and optionally other therapeutic agents. Such agents may be presented or encased within a suitable polymeric capsule dosage form comprises non-animal derived materials.

According to a further aspect of the invention there is provided a composition according to the invention for use in the treatment of vitamin $D_3$ deficiency or conditions in a subject that result from vitamin $D_3$ deficiency.

In a preferred embodiment of the invention said condition is osteoporosis.

In an alternative preferred embodiment of the invention said condition is osteomalacia.

In a further alternative preferred embodiment of the invention said condition is rickets.

In a preferred embodiment of the invention said condition is cancer.

In a further preferred embodiment of the invention said condition is diabetes.

In a preferred embodiment of the invention said condition is multiple sclerosis.

In a preferred embodiment of the invention said condition is depression.

In a preferred embodiment of the invention said subject is an elderly subject.

In an alternative preferred embodiment of the invention said subject is a paediatric subject.

Typically an elderly subject is one older than 60 years of age. A paediatric subject includes neonates (0-28 days old), infants (1 - 24 months old), young children (2 - 6 years old) and prepubescent [7-14 years old]. An elderly subject includes those over about the age of 60 years old.

We disclose a process for the manufacture of a high oral dosage form of vitamin $D_3$ containing no animal derived materials comprising:

> i) providing a soluble wax preparation comprising vitamin $D_3$ to provide a soluble preparation; and
>
> ii) dispensing the soluble preparation into capsules manufactured from cellulosic based material under non-oxidizing conditions.

We disclose that the wax preparation comprises a Poloxamer; preferably Poloxamer 188.

We disclose that cellulosic material is HPMC or similar.

We disclose an oral dosage form of vitamin $D_3$ obtained or obtainable by the process according to the invention.

We disclose a process for the manufacture of a high oral dosage form of vitamin $D_3$ containing no animal derived materials comprising:

> i) providing an oil based excipient comprising vitamin $D_3$ to provide a soluble preparation; and
>
> ii) dispensing the soluble preparation into capsules manufactured from cellulosic based material under non-oxidizing conditions.

We disclose that the oil based excipient is plant derived oil.

We disclose that the oil comprises coconut oil and palm kernel oil.

According to an aspect of the invention there is provided a process for the manufacture of a high oral dosage form of vitamin $D_3$ containing no animal derived materials comprising:

> i) providing an oil based excipient comprising: vitamin $D_3$, a mixture of caprylic/capric triglycerides, butylated hydroxyanisole and colloidal silicon dioxide to provide a soluble preparation; and
>
> ii) dispensing the soluble preparation into capsules manufactured from cellulosic based material under non-oxidizing conditions wherein said vitamin $D_3$ is 025%-1.0% w/w of said preparation.

In a preferred method of the invention said oil based excipient is Migyol 812N.

Preferably said viscosity modifying agent is colloidal silicon dioxide, for example Aerosil® 200.

In a preferred method of the invention colloidal silicon dioxide, for example Aerosil® 200, is provided at 0.5-5% w/w; preferably 2-4%w/w silicon dioxide.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

An embodiment of the invention will now be described by example only and with reference to the following tables:

**Materials and Methods**

**Manufacturing method for Vitamin D3 Formulations**

Method for preparing Vitamin D3 (Cholecalciferol) Poloxamer Formulations (FORMULATION 1-5)

a. Dispense the Poloxamer 188 into a suitable container.

b. Heat the container to approximately 65°C to melt the Poloxamer 188.

c. Dispense the Vitamin D3 into the same container and 'wet' the active powder by mixing with an overhead mixer to form a clear solution, maintain the solution in the molten state at 65°C.

d. Fill the solution from (c) into HPMC capsules using a suitable thermostatically controlled capsule filling machine. Maintain the solution in the molten state at 65°C.

e. Filling is to be conducted under nitrogen.
Method for preparing Vitamin D3 (Cholecalciferol) Miglyol 812N/BHA Formulations (FORMULATION 6-10)

f. Dispense the Miglyol 812N into a suitable container.

g. Dispense the Vitamin D3 into the same container and 'wet' the active powder by mixing with an overhead mixer to form a clear solution.

h. Dispense the BHA into the Vitamin D3 Miglyol 812N solution. Apply low shear mixing until a visibly homogenous solution is achieved. Degas the solution if required.

i. Fill the solution from (c) into HPMC capsules either by hand or using a suitable capsule filling machine.

j. Filling is to be conducted under nitrogen.

**Chemical compatibility for Vitamin D3 in a range of pharmaceutically acceptable oil and thermo-softening (waxy) excipients**

Binary/tertiary mix samples were prepared by dispensing approximately 0.25 g of Vitamin D3 (2.5% w/w concentration) into approximately 9.75 g of excipient(s). Thermo-softening excipients were preheated till fully molten before the preparation of samples. All mixes were mixed with a spatula for up to 5 minutes until a visibly homogenous mix was achieved. The mixing and flow characteristics of the mixes were assessed during the mixing process. Samples were placed on stability and assayed at T0 and after 4-weeks (T4) under accelerated storage conditions at 40°C/75%RH to accelerate product ageing. Analysis of the content of Vitamin D3 in the binary/tertiary mixes was conducted using HPLC with initial extraction from methanol. The HPLC conditions are as follows: Luna column, 5um, C18 100A, 150mm x 6 mm maintained at 25°C; gradient profile 30%A/70%B to 0%A/100% in 10 minutes - where A is methanol and B is methanol/ acetonitrile/tetrahydrofuran in a ratio of 65:20:15; flow rate at 1.0ml/min and detection via UV at 265nm.

**Stability for Vitamin D3 Formulations under ambient conditions**

Vitamin D (Cholecalciferol) Poloxamer 188 and Miglyol 812N/BHA formulated products, nominal dose strength: 100,000 IU (FORMULATIONS 5 and 10) were evaluated on stability at ambient temperature for 10-weeks. The analysis of the content of vitamin $D_3$ in the binary/tertiary mixes was conducted using HPLC with initial extraction from methanol. The HPLC conditions are as follows: Luna column, 5um, C18 100A, 150mm x 6 mm maintained at 25°C; gradient profile 30%A/70%B to 0%A/100% in 10 minutes - where A is methanol and B is methanol/acetonitrile/tetrahydrofuran in a ratio of 65:20:15; flow rate at 1.0ml/min and detection via UV at 265nm. The analysis was conducted by evaluating the increase in the impurity profile for vitamin $D_3$ as a function of storage time. The key (degradant) impurities monitored included those at relative retention time (RRT): 0.54, 0.63, 0.72, 0.82 and 0.86.

**Disintegration and dissolution characteristics for Vitamin D3 Formulations**

The disintegration and dissolution of Vitamin D (Cholecalciferol) Poloxamer 188 and Miglyol 812N/BHT formulated products, nominal dose strengths: 20,000 IU and 100,000 IU (FORMULATIONS 1, 5 and 6) were evaluated using the Pharmacopeial Disintegration tester. Each formulation was placed into the disintegration tester, with the water bath maintained at 37°C. The time for initial breach of the outer capsule and total time for disintegration and dissolution of the inner contents were recorded.

### Example 1

A summary of the compatibility data for vitamin $D_3$ in a range of pharmaceutically acceptable oils and thermo-softening polymer is shown in Table 1. Analysis of the binary/tertiary mixes shows that vitamin $D_3$ is subject to rapid chemical degradation in most oil-based excipients. Protection against the chemical degradation process can only be afforded by immobilising vitamin $D_3$ in the formulation matrix with the use of a thermo-softening polymer such as Poloxamer 188. Alternatively, the use of a chemical stability modifying additive such as butylated hydroxyannisole (BHA) or butylated hydroxytolouene (BHT) can produce a significant enhancement in chemical stability.

**Table 1**

| Binary/Tertiary Excipient | Initial Assay (%, T0) | Assay (%, T4 weeks at 40°C/75%RH) | Change* (%) |
|---|---|---|---|
| Miglyol 812N | 103.6 | 56.7 | -45 |
| Tween 20 | 101.6 | 79.8 | -21 |
| α-Tocopherol | 102.7 | 71.5 | -30 |
| Miglyol 812N/ Aerosil (2%) | 104.8 | 51.3 | -51 |
| TPGS | 95.3 | 29.8 | -69 |
| Gelucire 44/14 | 99.8 | 21.1 | -79 |
| Poloxamer 188 | 101.6 | 96.8 | -5 |
| Miglyol 812N/ BHA (2%) | 104.5 | 98.7 | -6 |
| Miglyol 812N/ BHT (2%) | 103.2 | 97.4 | -6 |
| * % change = ((T4 assay – Initial assay)/initial assay) x 100 | | | |

### Example 2

A summary of the stability data for FORMULATIONS 5 and 10 are shown Tables 2 and 3 respectively. It may be observed that consistent with the accelerated compatibility data presented in Table 1, Vitamin $D_3$ formulations containing Poloxamer 188 or Miglyol 812N/BHT show excellent stability profile, with limited increases in the degradant profile, following storage at ambient conditions after 10-weeks.

**Table 2: Stability profile for FORMULATION 5 at ambient temperature**

| Time-point (weeks) | RRT 0.54 | RRT 0.63 | RRT 0.72 | RRT 0.82 | RRT 0.86 |
|---|---|---|---|---|---|
| 0 | 0.10 | 0.04 | 0.03 | 0.03 | 0.04 |
| 4 | 0.08 | 0.04 | 0.05 | 0.06 | 0.08 |
| 10 | 0.11 | 0.04 | 0.04 | 0.07 | 0.08 |

**Table 3: Stability profile for FORMULATION 10 at ambient temperature**

| Time-point (weeks) | RRT 0.54 | RRT 0.63 | RRT 0.72 | RRT 0.82 | RRT 0.86 |
|---|---|---|---|---|---|
| 0 | 0.10 | 0.04 | 0.04 | 0.06 | 0.06 |
| 4 | 0.09 | 0.04 | 0.04 | 0.06 | 0.06 |
| 10 | 0.11 | 0.04 | 0.04 | 0.06 | 0.06 |

### Example 3

A summary of the disintegration and dissolution data and related observations for Vitamin D (Cholecalciferol) FORMULATIONS 1, 5 and 6 are shown in Table 4. These formulations passed the Pharmacopeial disintegration test (Ph.Eur. General Monograph for Hard Capsules) with a disintegration time of less than 30 minutes.

**Table 4: Disintegration and dissolution characteristics for Vitamin D (Cholecalciferol) FORMULATIONS 1. 5 and 6**

| | FORMULATION 1 (20,000 IU) | FORMULATION 5 (100,000 IU) | FORMULATION 6 (20,000 IU) |
|---|---|---|---|
| Breach time | 6 min | 9 min | 6 min |
| Time to full disintegration | 17 min | 28 min | 8 min |
| Observations | Total erosion by 17 min | Total erosion by 28 min | Instantly released and dispersed |

## Example 4

A viscosity modifying agent such as colloidal silicon dioxide [e.g. Aerosil® 200] is included to aid capsule filling. Aerosil 200 is added - 0.5-5% w/w; preferably 2-4% w/w or at about 2.44% w/w.

## Example 5

Long-term stability evaluation has been conducted with Formulations 11 and 12. Results are shown in Tables 5 and 6 respectively. Over the 36-week stability evaluation period, it can be noted that there was no significant change to the assay content for the formulations. In addition, the level of the putative impurity A (as defined within the British Pharmacopeia, 2010) remains essentially unchanged for both formulations 11 and 12.

**Table 5: Stability profile for FORMULATION 11 following long-term storage under ambient temperature condition**

| Time-points | Assay | RRT0.6 (Impurity A*) |
|---|---|---|
| 0 | 103% | 0.1% |
| 2 | 103% | 0.1% |
| 4 | 101% | 0.1% |
| 8 | 106% | 0.1% |
| 12 | 102% | 0.1% |
| 16 | 101% | 0.1% |
| 36 | 98% | LOQ |
| **Notes:** *Impurity A is defined within the British Pharmacopeia (BP), 2010 LOQ-limit of quantification | | |

**Table 6: Stability profile for FORMULATION 12 following long-term storage under ambient temperature conditions**

| Time-points (weeks) | Assay | RRT0.6 (Impurity A*) |
|---|---|---|
| 0 | 102% | 0.1% |
| 2 | 101% | 0.1% |
| 4 | 102% | 0.1% |
| 8 | 106% | 0.1% |
| 12 | 104% | 0.1% |
| 16 | 101% | 0.1% |
| 36 | 100% | LOQ |
| **Notes:** *Impurity A is defined within the British Pharmacopeia (BP), 2010 LOQ-limit of quantification | | |

Poloxamer 188 Vitamin D3 formulations

**FORMULATION 1:** Vitamin D3 (Cholecalciferol) Poloxamer 188 formulation, 20,000 IU

| Component | Supplier | mg per capsule | %w/w |
|---|---|---|---|
| Vitamin D3 | DSM Nutritional product | 0.50 | 0.26 |
| Poloxamer 188 | BASF Univar | 189.50 | 99.74 |
| HPMC Capsule | Capsugel | (Size 3) | - |
| **Total** | | 190.00 | 100.00 |

**FORMULATION 2:** Vitamin D3 (Cholecalciferol) Poloxamer 188 formulation, 50,000 IU

| Component | Supplier | mg per capsule | %w/w |
|---|---|---|---|
| Vitamin D3 | DSM Nutritional product | 1.25 | 0.26 |
| Poloxamer 188 | BASF Univar | 473.75 | 99.74 |
| HPMC Capsule | Capsugel | (Size 0) | - |
| **Total** | | 475.00 | 100.00 |

**FORMULATION 3:** Vitamin D3 (Cholecalciferol) Poloxamer 188 formulation, 100,000 IU

| Component | Supplier | mg per capsule | %w/w |
|---|---|---|---|
| Vitamin D3 | DSM Nutritional product | 2.5 | 0.26 |
| Poloxamer 188 | BASF Univar | 947.5 | 99.74 |
| HPMC Capsule | Capsugel | (Size 00EL) | - |
| **Total** | | 950.0 | 100.00 |

**FORMULATION 4:** Vitamin D3 (Cholecalciferol) Poloxamer 188 formulation, 50,000 IU

| Component | Supplier | mg per capsule | %w/w |
|---|---|---|---|
| Vitamin D3 | DSM Nutritional product | 1.25 | 0.50 |
| Poloxamer 188 | BASF Univar | 248.75 | 99.50 |
| HPMC Capsule | Capsugel | (Size 2) | - |
| **Total** | | 250 | 100.00 |

**FORMULATION 5:** Vitamin D3 (Cholecalciferol) Poloxamer 188 formulation, 100,000 IU

| Component | Supplier | mg per capsule | %w/w |
|---|---|---|---|
| Vitamin D3 | DSM Nutritional product | 2.5 | 1.00 |
| Poloxamer 188 | BASF Univar | 247.5 | 99.00 |
| HPMC Capsule | Capsugel | (Size 2) | - |
| **Total** | | 250.0 | 100.00 |

Miglyol 812N/BHA Vitamin D3 formulations

**FORMULATION 6:** Vitamin D3 (Cholecalciferol) Miglyol 812N/BHA formulation, 20,000 IU

| Component | Supplier | mg per capsule | %w/w |
|---|---|---|---|
| Vitamin D3 | DSM Nutritional product | 0.50 | 0.25 |
| Miglyol 812N | S Black | 199.40 | 99.70 |
| Butylated hydroxyanisole | Sigma-Aldrich | 0.10 | 0.05 |
| HPMC Capsule | Capsugel | (Size 3) | - |
| | Total | 200.00 | 100.00 |

**FORMULATION 7:** Vitamin D3 (Cholecalciferol) Miglyol 812N/BHA formulation, 50,000 IU

| Component | Supplier | mg per capsule | %w/w |
|---|---|---|---|
| Vitamin D3 | DSM Nutritional product | 1.25 | 0.25 |
| Miglyol 812N | S Black | 498.50 | 99.70 |
| Butylated hydroxyanisole | Sigma-Aldrich | 0.25 | 0.05 |
| HPMC Capsule | Capsugel | (Size 0) | - |
| | Total | 500.00 | 100.00 |

**FORMULATION 8:** Vitamin D3 (Cholecalciferol) Miglyol 812N/BHA formulation, 100,000 IU

| Component | Supplier | mg per capsule | %w/w |
|---|---|---|---|
| Vitamin D3 | DSM Nutritional product | 2.50 | 0.25 |
| Miglyol 812N | S Black | 997.00 | 99.70 |
| Butylated hydroxyanisole | Sigma-Aldrich | 0.50 | 0.05 |
| HPMC Capsule | Capsugel | (Size 00EL) | - |
| | Total | 1000.00 | 100.00 |

**FORMULATION 9:** Vitamin D3 (Cholecalciferol) Miglyol 812N/BHA formulation, 50,000 IU

| Component | Supplier | mg per capsule | %w/w |
|---|---|---|---|
| Vitamin D3 | DSM Nutritional product | 1.25 | 0.50 |
| Miglyol 812N | S Black | 248.62 | 99.70 |
| Butylated hydroxyanisole | Sigma-Aldrich | 0.13 | 0.05 |
| HPMC Capsule | Capsugel | (Size 2) | - |
| | Total | 250.00 | 100.00 |

**FORMULATION 10:** Vitamin D3 (Cholecalciferol) Miglyol 812N/BHA formulation, 100,000 IU

| Component | Supplier/grade | mg per capsule | %w/w |
|---|---|---|---|
| Vitamin D3 | DSM Nutritional product | 2.50 | 1.00 |
| Miglyol 812N | S Black | 247.37 | 98.95 |

(continued)

| Component | Supplier/grade | mg per capsule | %w/w |
|---|---|---|---|
| Butylated hydroxyanisole | Sigma-Aldrich | 0.13 | 0.05 |
| HPMC Capsule | Capsugel | (Size 2) | - |
| | Total | 250.00 | 100.00 |

**FORMULATION 11** Vitamin D3 (Cholecalciferol) Miglyol 812N/BHA/Aerosil formulation, 20,000 IU

| Component | Supplier | mg per capsule | %w/w |
|---|---|---|---|
| Vitamin D3 | DSM Nutritional product | 0.50 | 0.24 |
| Miglyol 812N | S Black | 199.40 | 97.27 |
| Butylated hydroxyanisole | Sigma-Aldrich | 0.10 | 0.05 |
| Colloidal Silicon Dioxide (Aerosil 200) | Evonik Industries | 5.00 | 2.44 |
| HPMC Capsule | Capsugel | (Size 3) | - |
| | Total | 205.00 | 100.00 |

**FORMULATION 12** Vitamin D3 (Cholecalciferol) Miglyol 812N/BHA/Aerosil formulation, 100,000 IU

| Component | Supplier/grade | mg per capsule | %w/w |
|---|---|---|---|
| Vitamin D3 | DSM Nutritional product | 2.50 | 0.87 |
| Miglyol 812N | S Black | 277.36 | 96.64 |
| Butylated hydroxyanisole | Sigma-Aldrich | 0.14 | 0.05 |
| Colloidal Silicon Dioxide (Aerosil 200) | Evonik Industries | 7.00 | 2.44 |
| HPMC Capsule | Capsugel | (Size 2) | - |
| | Total | 287.00 | 100.00 |

**Claims**

1. A pharmaceutical composition adapted for oral delivery comprising: at least 0.5mg [20,000 IU] of vitamin $D_3$, a mixture of caprylic/capric triglyceride excipient that stabilizes vitamin $D_3$, butylated hydroxyanisole, colloidal silicon dioxide and an outer capsule consisting of cellulosic polymer derivatives wherein vitamin $D_3$ is 0.25-1.0% w/w of the composition.

2. The composition according to claim 1 wherein vitamin $D_3$ is provided at 0.5mg [20,000 IU], 1.0mg [40,000IU], 1.25mg [50,000 IU], 1.5mg [60,000IU], 1.75mg [70,000 IU], 2mg [80,000IU], 2.25mg [90,000 IU] or 2.5mg [100,000IU] vitamin $D_3$.

3. The composition according to claim 1 or 2 wherein said outer capsule comprises HMPC.

4. A composition according to any one of claims 1 to 3 for use in the treatment of vitamin D deficiency or conditions that result from vitamin D deficiency.

5. The composition for use according to claim 4, wherein the condition is selected from the group consisting: osteoporosis, osteomalacia, rickets, cancer, diabetes, multiple sclerosis or depression.

6. A process for the manufacture of a high oral dosage form of vitamin $D_3$ containing no animal derived materials comprising:

i) providing an oil based excipient comprising: vitamin $D_3$, a mixture of caprylic/capric triglycerides, butylated hydroxyanisole and colloidal silicon dioxide to provide a soluble preparation; and
ii) dispensing the soluble preparation into capsules manufactured from cellulosic based material under non-oxidizing conditions wherein said vitamin $D_3$ is 0.25%-1.0% w/w of said preparation.

7. The process according to claim 6 wherein said cellulosic based material is HMPC.

**Patentansprüche**

1. Pharmazeutische Zusammensetzung, die zur oralen Verabreichung geeignet ist, umfassend: mindestens 0,5 mg [20.000 IU] Vitamin $D_3$, ein Vitamin $D_3$ stabilisierendes Gemisch aus Capryl-/Caprintriglycerid-Hilfsstoff, butyliertes Hydroxyanisol, kolloidales Siliziumdioxid und eine aus Cellulosepolymerderivaten bestehende Außenkapsel, wobei Vitamin $D_3$ 0,25-1,0 Gew.-% der Zusammensetzung ausmacht.

2. Zusammensetzung nach Anspruch 1, wobei Vitamin $D_3$ mit 0,5 mg [20.000 IU], 1,0 mg [40.000 IU], 1,25 mg [50.000 IU], 1,5 mg [60.000 IU], 1,75 mg [70.000 IU], 2 mg [80.000 IU], 2,25 mg [90.000 IU] oder 2,5 mg [100.000 IU] Vitamin $D_3$ bereitgestellt ist.

3. Zusammensetzung nach Anspruch 1 oder 2, wobei die Außenkapsel HMPC umfasst.

4. Zusammensetzung nach einem der Ansprüche 1 bis 3 zur Verwendung bei der Behandlung von Vitamin-D-Mangel oder aus Vitamin-D-Mangel resultierenden Zuständen.

5. Zusammensetzung zur Verwendung nach Anspruch 4, wobei der Zustand ausgewählt ist aus der Gruppe bestehend aus: Osteoporose, Osteomalazie, Rachitis, Krebs, Diabetes, multipler Sklerose oder Depression.

6. Verfahren zur Herstellung einer hohen oralen Dosierungsform von Vitamin $D_3$, die keine von Tieren abgeleiteten Materialien enthält, umfassend:

i) Bereitstellen eines Hilfsstoffs auf Ölbasis, umfassend: Vitamin $D_3$, ein Gemisch aus Capryl-/Caprintriglyceriden, butyliertes Hydroxyanisol und kolloidales Siliziumdioxid, zum Bereitstellen eines löslichen Präparats; und
ii) Verteilen des löslichen Präparats in aus einem Material auf Cellulosebasis hergestellten Kapseln bei nichtoxidierenden Bedingungen, wobei das Vitamin $D_3$ 0,25-1,0 Gew.-% des Präparats ausmacht.

7. Verfahren nach Anspruch 6, wobei das Material auf Cellulosebasis HMPC ist.

**Revendications**

1. Composition pharmaceutique adaptée pour une administration orale, comprenant : au moins 0,5 mg [20 000 UI] de vitamine $D_3$, un mélange d'excipient triglycéride caprylique/caprique qui stabilise la vitamine $D_3$, l'hydroxyanisole butylé, le dioxyde de silicone colloïde et une capsule externe composée de dérivés de polymère cellulosique dans laquelle la vitamine $D_3$ représente 0,25 à 1,0 % p/p de la composition.

2. Composition selon la revendication 1, dans laquelle la vitamine $D_3$ est administrée à 0,5 mg [20 000 UI], 1,0 mg [40 000 UI], 1,25 mg [50 000 UI], 1,5 mg [60 000 UI], 1,75 mg [70 000 UI], 2 mg [80 000 UI], 2,25 mg [90 000 UI] ou 2,5 mg [100 000 UI] de vitamine $D_3$.

3. Composition selon la revendication 1 ou 2, dans laquelle la capsule externe comprend du HMPC.

4. Composition selon l'une quelconque des revendications 1 à 3, pour une utilisation dans le traitement d'une déficience en vitamine D ou des pathologies qui résultent d'une déficience en vitamine D.

5. Composition pour une utilisation selon la revendication 4, dans laquelle la pathologie est choisie dans le groupe composé : de l'ostéoporose, de l'ostéomalacie, du rachitisme, du cancer, du diabète, de la sclérose en plaques ou de la dépression.

**6.** Procédé pour la fabrication d'une forme galénique orale élevée de la vitamine $D_3$ ne contenant aucun produit dérivé des animaux, comprenant :

i) l'utilisation d'un excipient à base d'huile comprenant : de la vitamine $D_3$, d'un mélange de triglycérides caprylique/caprique, de l'hydroxyanisole butylé et du dioxyde de silicone colloïde pour former une préparation soluble ; et
ii) la distribution de la préparation soluble dans des capsules fabriquées à partir d'un matériau à base de cellulose dans des conditions non-oxydantes dans lesquelles ladite vitamine $D_3$ représente 0,25 % à 1,0 % p/p de ladite préparation.

**7.** Procédé selon la revendication 6, dans lequel ledit matériau à base de cellulose est l'HMPC.

**EP 2 680 826 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009098295 A
- WO 2009009134 A
- US 20090196862 A1
- US 20030191093 A1, Chen
- WO 2010111397 A

**Non-patent literature cited in the description**

- **ARMAS et al.** *JCEM,* 2004, vol. 98 (11